(19) Europäisches Patentamt / European Patent Office / Office européen des brevets

(11) **EP 2 984 988 B1**

## (12) EUROPÄISCHE PATENTSCHRIFT

(45) Veröffentlichungstag und Bekanntmachung des Hinweises auf die Patenterteilung:
**08.06.2022 Patentblatt 2022/23**

(51) Internationale Patentklassifikation (IPC):
**A61B 5/145** (2006.01)   **A61B 5/1455** (2006.01)
**A61M 1/36** (2006.01)   **G01N 33/49** (2006.01)

(21) Anmeldenummer: **15183364.7**

(22) Anmeldetag: **20.07.2012**

(52) Gemeinsame Patentklassifikation (CPC):
**G01N 33/49; A61B 5/14557; A61M 1/367;**
**G01N 21/05; G01N 21/31;** A61M 2205/14;
A61M 2205/3306; A61M 2230/20; G01N 2021/0378;
G01N 2021/3144

(54) **VORRICHTUNG UND VERFAHREN ZUR ERKENNUNG EINER IN DIE AUFNAHMEELEMENTE EINER EINSPANNEINHEIT ZUM EINSPANNEN EINER SCHLAUCHLEITUNG EINGELEGTEN SCHLAUCHLEITUNG**

DEVICE AND METHOD FOR DETECTING A HOSE LINE LAID IN THE RECEIVING ELEMENT OF A TENSIONING UNIT FOR TENSIONING A HOSE LINE

DISPOSITIF ET PROCEDE DE RECONNAISSANCE D'UNE CONDUITE FLEXIBLE INSEREE DANS DES ELEMENTS DE RECEPTION D'UNE UNITE DE SERRAGE DESTINEE A SERRER UNE CONDUITE FLEXIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.07.2011 DE 102011108050**
**21.07.2011 US 201161510104 P**

(43) Veröffentlichungstag der Anmeldung:
**17.02.2016 Patentblatt 2016/07**

(62) Dokumentnummer(n) der früheren Anmeldung(en) nach Art. 76 EPÜ:
**12738045.9 / 2 734 111**

(73) Patentinhaber: Fresenius Medical Care
**Deutschland GmbH**
**61352 Bad Homburg (DE)**

(72) Erfinder:
• **Zhang, Wei**
**97464 Niederwerrn (DE)**

• **Schulte, Elke**
**97422 Schweinfurt (DE)**
• **Kaiser, Martin**
**97437 Hassfurt (DE)**
• **Müller, Carsten**
**97502 Euerbach (DE)**
• **Klein, Thomas**
**97737 Gemünden am Main (DE)**
• **Zerle, Stefan**
**86916 Kaufering (DE)**
• **Bardorz, Christoph**
**97228 Rottendorf (DE)**
• **Stühler, Stefan**
**97453 Schonungen (DE)**

(74) Vertreter: **Oppermann, Frank**
**OANDO Oppermann & Oppermann LLP**
**Wilhelminenstrasse 1a**
**65193 Wiesbaden (DE)**

(56) Entgegenhaltungen:
**WO-A1-2004/057313    WO-A1-2008/000433**
**DE-A1- 19 530 969    US-A- 5 372 136**

**Beschreibung**

Die Erfindung betrifft eine Vorrichtung zum Einspannen einer Schlauchleitung mit einer Einspanneinheit insbesondere einer Vorrichtung zur Bestimmung der Konzentration eines Bestandteils von Blut in einer Schlauchleitung, insbesondere einer Schlauchleitung eines extrakorporalen Blutkreislaufs einer extrakorporalen Blutbehandlungsvorrichtung. Darüber hinaus betrifft die Erfindung ein Verfahren zur Erkennung einer Schlauchleitung, insbesondere einer Schlauchleitung eines extrakorporalen Blutkreislaufs einer extrakorporalen Blutbehandlungsvorrichtung, in einer Einspanneinheit insbesondere einer Vorrichtung zur Bestimmung der Konzentration eines Blutbestandteils in einer Schlauchleitung.

Zur Bestimmung der Konzentration von bestimmten Bestandteilen im Blut eines Patienten sind verschiedene Verfahren bekannt. Im Stand der Technik sind Verfahren zur Messung der Konzentration von Blutbestandteilen bekannt, die eine Entnahme einer Blutprobe voraussetzen. Es sind aber auch Messverfahren bekannt, bei denen die Konzentration von Blutbestandteilen gemessen wird, während das Blut durch die Schlauchleitung strömt. Diese Verfahren finden insbesondere dann Anwendung, wenn bei einer extrakorporalen Blutbehandlung das Blut in der Schlauchleitung eines extrakorporalen Blutkreislaufs fließt.

Die WO 2008/00433 A1 beschreibt eine Vorrichtung zur Bestimmung der Konzentration von bestimmten Blutbestandteilen in einer mit Blut gefüllten im Wesentlichen transparenten Schlauchleitung eines extrakorporalen Blutkreislaufs. Die bekannte Vorrichtung erlaubt insbesondere die Bestimmung der Hämoglobinkonzentration und des Anteils der roten Blutkörperchen (Erythrozyten) am Gesamtvolumen des Bluts. Während der Messung ist die Schlauchleitung zwischen zwei parallelen, ebenen Anlageflächen eingespannt, so dass sich der Schlauch an den einander gegenüberliegenden Seiten verformt. Mit einem Lichtemitter wird Licht einer bestimmten Wellenlänge durch die transparente Schlauchleitung in das Blut eingekoppelt, während mit einem Lichtdetektor

das gestreute oder transmittierte Licht gemessen wird. Der Hämatokrit wird dann aus dem Verhältnis der Intensität des in das Blut eintretenden und aus dem Blut austretenden Lichts bestimmt.

Aus der EP 1 579 196 B1 ist eine Vorrichtung zur Bestimmung von Blutbestandteilen bekannt, die über eine Einspanneinheit zum Einspannen der Schlauchleitung und eine Messeinheit verfügt. Die Einspanneinheit ist derart ausgebildet, dass die eingespannte Schlauchleitung einen quadratischen Querschnitt hat. Die Messeinheit verfügt über mehrere Lichtemitter und Lichtdetektoren, die um den Umfang der Schlauchleitung angeordnet sind. Die Lichtemitter und Lichtdetektoren sind derart angeordnet, dass die Lichtemitter in einer anderen Ebene als die Lichtdetektoren liegen, so dass sich Lichtemitter und Lichtdetektoren nicht einander gegenüberliegen. Zur Messung der Blutparameter wird die Schlauchleitung in der Einspanneinheit verformt. Dabei ist darauf zu achten, dass die Schlauchleitung sich nicht in der Einspanneinheit verklemmt.

Darüber hinaus wird angestrebt, dass reproduzierbare Messungen der Blutparameter für den Anwender ohne großen Aufwand möglich sind. Eine Automatisierung des Messablaufs setzt auch die Erkennung der in die Einspanneinheit eingelegten Schlauchleitungen voraus. Auch spielt bei der Messung in der Praxis die Leistung und Lebensdauer der als Lichtemitter verwendeten Leuchtdioden (LEDs) eine Rolle.

Die US 5 372 136 A beschreibt eine Vorrichtung zur Bestimmung des Hämatokrit, die über eine als Ohrclip ausgebildete Einspanneinheit verfügt. Die Einspanneinheit weist zwei Klemmbacken zum Einklemmen des Ohrläppchens auf, um einen pulsierenden Blutfluss zu erzeugen. Die eine Klemmbacke kann gegenüber der anderen Klemmbacke mit einer elektromotorisch angetriebenen Betätigungseinrichtung verfahren werden, so dass auf das Ohrläppchen eine Anpresskraft aufgebracht wird. Darüber hinaus verfügt die Vorrichtung zur Bestimmung des Hämatokrit über eine Messeinheit mit einem auf der einen Seite des Ohrläppchens angeordneten Sender zum Einkoppeln von elektromagnetischer

Strahlung und einen auf der anderen Seite des Ohrläppchens angeordneten Empfänger zum Auskoppeln von elektromagnetischer Strahlung.

Der Erfindung liegt die Aufgabe zugrunde, eine Vorrichtung zum Einspannen einer Schlauchleitung zu schaffen, die eine Erkennung einer in die Aufnahmeelemente einer Einspanneinheit der Vorrichtung zum Einspannen der Schlauchleitung eingelegten Schlauchleitung erlaubt, insbesondere eine Vorrichtung zur Bestimmung der Konzentration von Blutparametern in einer Schlauchleitung zu schaffen, die einen automatisierten Messablauf mit hoher Messgenauigkeit und die Erkennung einer in die Aufnahmeelemente der Einspanneinheit der Vorrichtung zur Bestimmung der Konzentration von Blutparametern eingelegten Schlauchleitung erlaubt.

Eine weitere Aufgabe der Erfindung ist ein Verfahren zur Erkennung einer Schlauchleitung in einer Einspanneinheit einer Vorrichtung zum Einspannen einer Schlauchleitung insbesondere einer Vorrichtung zur Bestimmung der Konzentration eines Blutbestandteils anzugeben.

Die erfindungsgemäße Vorrichtung zur Bestimmung der Konzentration eines Bestandteils von Blut in einer Schlauchleitung, insbesondere der Schlauchleitung eines extrakorporalen Blutkreislaufs, zeichnet sich dadurch aus, dass die Einspanneinheit einen Betätigungsmechanismus aufweist, der derart ausgebildet ist, dass unter Aufbringung einer Spannkraft ein erstes und zweites Aufnahmeelement aus einer ersten die Schlauchleitung freigebenden Stellung in eine zweite die Schlauchleitung einspannende Stellung gegeneinander bewegbar sind, wobei der Antrieb des Betätigungsmechanismus mit einem Elektromotor erfolgt. Dadurch wird eine voll automatisch arbeitende Einspanneinheit geschaffen, in die der Benutzer die Schlauchleitung nur einzulegen braucht.

Da die beiden Aufnahmeelemente für die Schlauchleitung elektromotorisch gegeneinander verfahren werden, wird die erforderliche Spannkraft gleichmäßig aufgebracht. Der elektromotorische Antrieb erlaubt die Einstellung einer bestimmten Vorschubgeschwindigkeit, mit der die Aufnahmeelemente bewegt werden. Damit ist es möglich, die Schlauchleitung mit einer optimalen Vorschubgeschwindigkeit zusammen zu drücken, so dass die Schlauchleitung Gelegenheit hat, sich zwischen den Aufnahmeelementen exakt zu zentrieren. Dadurch wird ein Verklemmen der Schlauchleitung in Folge einer zu schnellen und nicht gleichförmigen Bewegung der Spannelemente vermieden.

Darüber hinaus zeichnet sich die erfindungsgemäße Vorrichtung zur Bestimmung der Konzentration von Blutbestandteilen durch eine Überwachungseinheit aus, die derart ausgebildet ist, dass die in die Aufnahmeelemente eingelegte Schlauchleitung erkennbar ist. Dadurch ist eine Automatisierung der Messung der Blutparameter möglich. Beispielsweise kann die Messung erst und nur dann gestartet werden, wenn die Schlauchleitung in die Einspanneinheit eingelegt ist. Damit kann die Leuchtdauer der als Lichtemitter verwendeten LEDs verringert werden, so dass sich die Lebensdauer des LEDs erhöht. Im Übrigen werden Fehlmessungen bei nicht eingelegter Schlauchleitung vermieden.

Bei der erfindungsgemäßen Vorrichtung wird zur Erkennung der Schlauchleitung in der Einspanneinheit der Motorstrom des Elektromotors zum Antrieb des Betätigungsmechanismus der Einspanneinheit gemessen, wobei auf der Grundlage der Veränderung des gemessenen Motorstroms die in die Aufnahmeelemente der Einspanneinheit eingelegte Schlauchleitung erkannt wird. Anstelle des Motorstroms des Elektromotors zum Antrieb des Betätigungsmechanismus kann auch jede mit dem Motorstrom korrelierende Größe, bspw. die Motorleistung, ausgewertet werden. Die Auswertung des Motorstroms zur Erkennung der Schlauchleitung kann in einer Rechen- und Auswerteinheit erfolgen, ohne dass weitere mechanische Komponenten erforderlich sind. Die Rechen- und Auswerteinheit der Überwachungseinheit kann Bestandteil der Rechen- und Auswerteinheit der Vorrichtung zur Bestimmung der Konzentration des Blutbestandteils sein. Beide Rechen- und Auswerteinheiten können auch Bestandteil der zentralen Steuereinheit oder Rechen- und Auswerteinheit einer extrakorporalen Blutbehandlungsvorrichtung sein.

Es hat sich gezeigt, dass der Motorstrom einen charakteristischen Verlauf hat, der davon abhängig ist, ob eine Schlauchleitung eingespannt wird oder nicht. Beim Schließen der Aufnahmeelemente zeigt sich ein deutlicher Anstieg des Motorstroms wesentlich früher, wenn die Schlauchleitung in die Aufnahmeelemente eingelegt ist. Auch ist bei eingelegter Schlauchleitung der Anstieg des Motorstroms deutlich steiler. Somit kann der zeitliche Verlauf des Motorstroms beim Schließen der Aufnahmeelemente als Kriterium für die Erkennung der Schlauchleitung herangezogen werden.

Bei einer besonders bevorzugten Ausführungsform verfügt die Überwachungseinheit über Mittel zum Messen der von den Aufnahmeelementen zurückgelegten Wegstrecke, wobei die Rechen- und Auswerteinheit der Überwachungseinheit derart ausgebildet ist, dass der Motorstrom in Abhängigkeit von der von den Aufnahmeelementen zurückgelegten Wegstrecke ausgewertet wird. Die Rechen- und Auswerteinheit ist bei der besonders bevorzugten Ausführungsform derart ausgebildet, dass das Integral des Motorstroms über eine vorgegebene von den Aufnahmeelementen zurückgelegte Wegstrecke mit einem vorgegebenen Grenzwert verglichen wird, wobei bei Überschreiten des Grenzwertes darauf geschlossen wird, dass in die Aufnahmeelemente eine Schlauchleitung eingelegt ist. Die Integration des Motorstroms braucht nicht kontinuierlich, sondern kann auch in diskreten Zeitintervallen erfolgen. Der Grenzwert kann in Abhängigkeit von dem Material der verwendeten Schlauchleitung vorgegeben werden, wobei für verschiedene Schlauchleitungen auch mehrere Grenzwerte vorgegeben werden können.

Eine besondere Ausführungsform der Einspanneinheit, die von eigener erfinderischer Bedeutung ist, zeichnet sich dadurch aus, dass das erste Aufnahmeelement zwei rechtwinklig zueinander stehende ebene Anlageflächen und das zweite Aufnahmeelement zwei rechtwinklig zueinander stehende ebene Anlageflächen aufweist, wobei das erste und zweite Aufnahmeelement auf einer Achse gegeneinander bewegbar sind, die mit den ebenen Anlageflächen des ersten und zweiten Aufnahmeelements einen Winkel von 45° einschließt. Dabei ist unerheblich, wie die beiden Aufnahmeelemente ausgebildet sind, solange die rechtwinklig zueinander stehenden ebenen Anlageflächen vorhanden sind.

Die Aufnahmeelemente mit den ebenen Anlageflächen verformen die Schlauchleitung beim Schließen der Einspanneinheit. Vorzugsweise weisen das erste und zweite Aufnahmeelement zu beiden Seiten der ebenen Anlageflächen halbzylindrische Anlageflächen auf. In dem Bereich der halbzylindrischen Anlageflächen wird die eingelegte Schlauchleitung nicht verformt.

Eine besonders bevorzugte Ausführungsform sieht zwischen den ebenen Anlageflächen und den halbzylindrischen Anlageflächen der beiden Aufnahmeelemente einen Übergangsabschnitt vor, der derart ausgebildet ist, dass die inneren ebenen Anlageflächen kontinuierlich in die äußeren halbzylindrischen Anlageflächen übergehen. Dabei ist unter einem kontinuierlichen Übergang jeder Übergang zu verstehen, der eine gleichmäßige Verformung der Schlauchleitung erlaubt, so dass der kreisförmige Querschnitt der Schlauchleitung gleichförmig in den quadratischen Querschnitt übergeht und die Schlauchleitung nicht geknickt wird.

Der gleichmäßige Übergang von dem kreisförmigen in den quadratischen Querschnitt hat nicht nur den Vorteil, dass die Schlauchleitung beim Schließen der Einspanneinheit geschont wird, sondern auch den Vorteil, dass turbulente Strömungen beim Übergang von dem runden zum quadratischen Leitungsquerschnitt vermieden werden. Dadurch kann die Länge des quadratischen Messkanals, in dem Lichtemitter und Lichtdetektoren angeordnet werden, möglichst gering gehalten werden. Eine Verkürzung des Messkanals hat wiederum kleine Schließkräfte zur Folge, was ebenfalls von Vorteil ist.

Die Messeinheit der Vorrichtung zur Bestimmung der Konzentration von Blutbestandteilen weist vorzugsweise mehrere Lichtemitter zum Einkoppeln von elektromagnetischer Strahlung durch die Schlauchleitung in das Blut und mehrere Lichtdetektoren zum Messen der durch die Schlauchleitung aus dem Blut austretenden elektromagnetischen Strahlung auf, die in dem ersten und zweiten Aufhahmeelement angeordnet sind. Vorzugsweise sind die Lichtemitter und Lichtdetektoren in Lichtaustritts- und -eintrittsöffnungen angeordnet, die in den ebenen Anlageflächen der Aufnahmeelemente vorgesehen sind. Damit sind die Lichtemitter und Lichtdetektoren in die Aufnahmeelemente integriert.

Bei einer bevorzugten Ausführungsform der Messeinheit, die von eigener erfinderischer Bedeutung ist, sind mindestens einem Lichtdetektor mindestens eine Gruppe von zwei Lichtemittern zugeordnet. Es können dem mindestens einen Lichtdetektor bspw. 2, 4, 6 oder 8 Lichtemitter zugeordnet werden. In der Praxis ist aber die Zuordnung von 2 Lichtemittern zu einem Lichtdetektor ausreichend.

Die Verwendung von zwei Lichtemittern anstelle nur eines Lichtmitters zum Einkoppeln von Licht in die Schlauchleitung hat den Vorteil, dass die Lichtausbeute der LEDs verdoppelt wird, während die Einflüsse der Schlauchleitung auf die von dem Licht zurückgelegte Strecke verringert wird.

Nachteilig ist, dass die Signalamplitude mit zunehmendem Abstand von Lichtemitter und Lichtdetektor abnimmt. Daher wird grundsätzlich eine Erhöhung der Intensität des eingekoppelten Lichts angestrebt. Dies schränkt aber die Auswahl der als Lichtemitter zur Verfügung stehenden Bauelemente stark ein. Ein geringerer Abstand von Lichtemittern und Lichtdetektoren, der eine Erhöhung der Intensität der Strahlung nicht erforderlich macht, beeinträchtigt hingegen die Genauigkeit der Messung. Dazu kommen Veränderungen der Schlauchwandstärke, die bei den bekannten Extrusionsprozessen zur Herstellung der Schlauchleitungen auf kurzen Abschnitten der Schlauchleitungen nicht auszuschließen sind. So hat der Schlauch nur in erster Näherung über seinen ganzen Umfang dieselbe Wandstärke.

Die Zusammenfassung von zwei auf einandergegenüberliegenden Seiten der Schlauchleitung angeordneten Lichtemittern zu einer Gruppe verdoppelt die Lichtausbeute. Da das Licht beider Lichtemitter auf unterschiedlichen Messstrecken ausgewertet wird, werden die Einflüsse der Schlauchleitung gemittelt, wodurch die Messgenauigkeit erhöht wird.

Die besondere Ausbildung der Aufhahmeelemente der Einspanneinheit mit den ebenen und zylindrischen Anlageflächen setzt nicht zwingend die besondere Ausbildung der Messeinheit und besondere Ausbildung der Einspanneinheit mit dem Betätigungsmechanismus voraus, der über einen elektromotorischen Antrieb verfügt. Auch die besondere Ausbildung der Messeinheit setzt nicht die besondere Ausbildung der Aufnahmeelemente der Einspanneinheit und die besondere Ausbildung des elektromotorisch angetriebenen Betätigungsmechanismus der Einspanneinheit voraus.

Im Folgenden wird ein Ausführungsbeispiel der Erfindung unter Bezugnahme auf die Zeichnungen näher erläutert.

Es zeigen:

Fig. 1 eine Vorrichtung zur extrakorporalen Blutbehandlung zusammen mit einer Vorrichtung zum Bestimmen der Konzentration eines Blutbestandteils in stark vereinfachter schematischer Darstellung,

Fig. 2A eine stark vereinfachte schematische Darstellung der Einspanneinheit und der Messeinheit der Vorrichtung zur Bestimmung der Konzentration eines Blutbestandteils, wobei die Anordnung der Lichtemitter und Lichtdetektoren in einer ersten Schnittebene gezeigt wird,

Fig. 2B die Einspanneinheit und Messeinheit in stark vereinfachter schematischer Darstellung, wobei die Anordnung der Lichtemitter und Lichtdetektoren in einer zweiten Schnittebene gezeigt wird, die von der ersten Schnittebene verschieden ist,

Fig. 3A das eine Aufnahmeelement der Einspanneinheit in perspektivischer Darstellung,

Fig. 3B das andere Aufnahmeelement der Einspanneinheit in perspektivischer Darstellung,

Fig. 4A den zeitlichen Verlauf des bei einer ersten Einspanneinheit gemessenen Motorstroms des Elektromotors für die elektromotorische Betätigung der Einspanneinheit,

Fig. 4B den zeitlichen Verlauf des bei einer zweiten Einspanneinheit gemessenen Motorstroms,

Fig. 4C den zeitlichen Verlauf des bei einer dritten Einspanneinheit gemessenen Motorstroms,

Fig. 5 eine Auswertung der Messergebnisse von den Fig. 4A - 4C,

Fig. 6 den Motorstrom in Abhängigkeit von dem Drehmoment,

Fig. 7 die Messergebnisse für sechs Einspanneinheiten der gleichen Bauart und unterschiedliche Schlauchleitungen,

Fig. 8A bis

Fig. 8D den Motorstrom I als Funktion der Motorstrecke x für die Einspanneinheiten der gleichen Bauart und die unterschiedlichen Schlauchtypen und

Fig. 9 eine Tabelle, in der das Integral, der maximale Strom und die Steigung der Stromanstiegsflanke für die Einspanneinheiten der gleichen Bauart und die unterschiedlichen Schlauchtypen eingetragen ist.

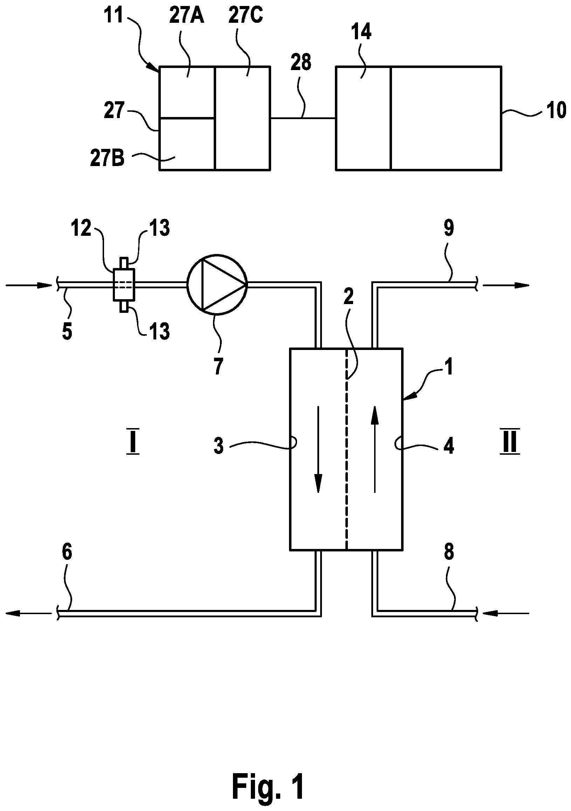

Fig. 1 zeigt nur die für die Erfindung wesentlichen Komponenten einer Vorrichtung zur extrakorporalen Blutbehandlung in stark vereinfachter schematischer Darstellung. Die extrakorporale Blutbehandlungsvorrichtung, bspw. Dialysevorrichtung, verfügt über einen Dialysator oder Filter 1, der durch eine semipermeable Membran 2 in eine Blutkammer 3 und eine Dialysierflüssigkeitskammer 4 unterteilt ist. Von dem Patienten führt eine arterielle Blutleitung 5 zu der Blutkammer 3, während von der Blutkammer 3 eine venöse Blutleitung 6 abgeht, die zu dem Patienten führt. Eine in der arteriellen Blutleitung 5 angeordnete Blutpumpe 7 fördert das Blut im extrakorporalen Blutkreislauf I. Das Dialysierflüssigkeitssystem II der Dialysevorrichtung ist nur andeutungsweise dargestellt. Es umfasst eine zu der Dialysierflüssigkeitskammer 4 führende Dialysierflüssigkeitszuführleitung 8 und eine von der Dialysierflüssigkeitskammer 4 abgehende Dialysierflüssigkeitsabführleitung 9. Bei der arteriellen und venösen Blutleitung 5, 6 handelt es sich um Schlauchleitungen, die für Licht zumindest teilweise durchlässig sind. Darüber hinaus verfügt die Blutbehandlungsvorrichtung über eine zentrale Steuereinheit 10, mit der die einzelnen Komponenten, bspw. die Blutpumpe 7 gesteuert werden.

Die Vorrichtung 11 zur Bestimmung der Konzentration von bestimmten Bestandteilen im Blut des Patienten kann Bestandteil der extrakorporalen Blutbehandlungsvorrichtung sein oder eine separate Baugruppe bilden. Wenn die Vorrichtung 11 Bestandteil der Blutbehandlungsvorrichtung ist, kann sie von den Komponenten Gebrauch machen, die in der Blutbehandlungsvorrichtung ohnehin vorhanden sind.

Die Vorrichtung 11 zum Bestimmen der Konzentration von Blutbestandteilen, insbesondere der Hämoglobinkonzentration (Hb), des Hämatokrit (Hkt) oder des relativen Blutvolumens (RBV) verfügt über eine in Fig. 1 nur andeutungsweise dargestellte Einspanneinheit 12 zur Aufnahme der Schlauchleitung, insbesondere der arteriellen Blutleitung 5, und eine Messeinheit 13 zum Einkoppeln von Licht in das durch die Blutleitung 5 strömende Blut und Messen des aus dem Blut austretenden Lichts. Die Messeinheit 13 wirkt mit einer Rechen- und Auswerteinheit 14 zusammen, die aus den Messwerten die Konzentration des Blutbestandteils bestimmt. Auf die Beschreibung der Auswertung der Messwerte zur Bestimmung der Blutbestandteile im Einzelnen wird verzichtet, da die Bestimmung der Konzentration des Blutbestandteils aus den Messwerten bekannt ist. Die Bestimmung der Blutbestandteile wird bspw. in der EP 1 579 196 B1 im Einzelnen beschrieben.

Bei dem vorliegenden Ausführungsbeispiel ist die Rechen- und Auswerteinheit 14 zum Bestimmen der Konzentration eines Blutbestandteils Bestandteil der zentralen Steuereinheit 10 oder Rechen- und Auswerteinheit der extrakorporalen Blutbehandlungsvorrichtung. Es können aber auch getrennte Einheiten vorgesehen sein.

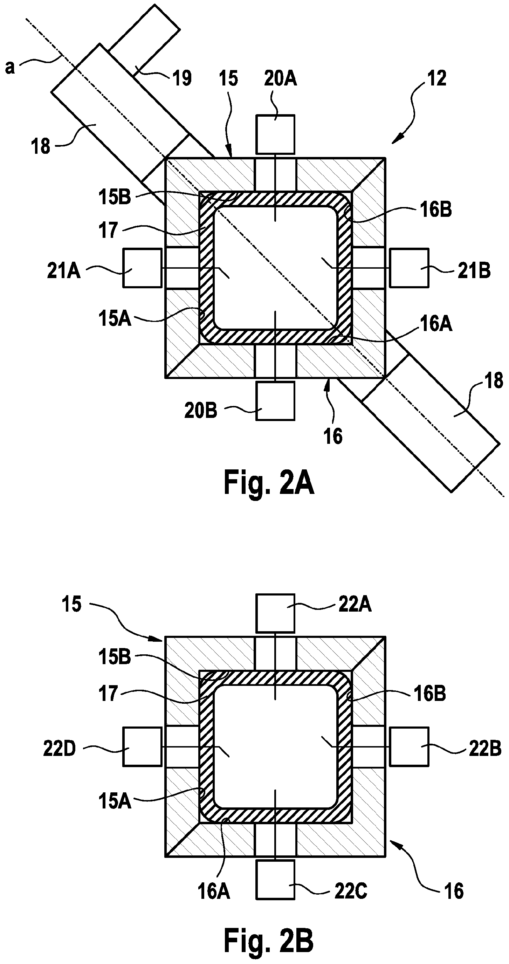

Die Fig. 2A und 2B zeigen in verschiedenen Schnittebenen und in stark vereinfachter schematischer Darstellung die Einspanneinheit 12 und die Messeinheit 13 der Vorrichtung 11.

Die Einspanneinheit 12 weist zwei Aufnahmeelemente 15, 16 auf, zwischen denen die Schlauchleitung 17 eingespannt wird, so dass die runde Schlauchleitung einen quadratischen Querschnitt erhält. Zum Einlegen der Schlauchleitung können die Aufnahmeelemente 15, 16 entlang einer Achse a gegeneinander verfahren werden. Zum Bewegen der Einspannelemente 15, 16 aus einer die Schlauchleitung 17 freigebenden Stellung in eine die Schlauchleitung einspannende Stellung dient ein Betätigungsmechanismus 18, der in den Fig. 2A und 2B nur schematisch dargestellt ist.

Die Aufnahmeelemente 15, 16 und der Betätigungsmechanismus 18 können unterschiedlich ausgebildet sein. Zum Antrieb des Betätigungsmechanismus 18 dient ein Elektromotor 19, der ebenfalls nur schematisch dargestellt ist. Wenn der Elektromotor in die eine oder andere Richtung dreht, werden die Aufnahmeelemente entlang der Achse a auf- und zugefahren. Die Fig. 2A und 2B zeigen die Einspanneinheit 12 in der geschlossenen Stellung.

Die Aufnahmeelemente 15, 16 weisen jeweils zwei ebene Anlageflächen 15A, 15B bzw. 16A, 16B auf, die jeweils einen rechten Winkel einschließen. Die Achse a, auf der die Aufnahmeelemente 15, 16 verfahren werden, schließen mit den ebenen Anlageflächen 15A, 15B bzw. 16A, 16B einen Winkel von 45° ein.

Die Messeinheit 13 verfügt über mehrere Lichtemitter 20A, 20B; 21A, 21B, die in einer ersten Ebene (Fig. 2A) um den Umfang der Schlauchleitung angeordnet sind. Die Achsen der benachbarten Lichtemitter schließen jeweils einen Winkel von 90° ein. Die Lichtdetektoren 22A, 22B, 22C, 22D sind in einer zweiten Ebene angeordnet, die von der ersten Ebene verschieden ist (Fig. 2B). Bei dem vorliegenden Ausführungsbeispiel weist die Messeinheit vier Lichtdetektoren auf, die um den Umfang der Schlauchleitung angeordnet sind. Die Lichtemitter und Lichtdetektoren sind Leuchtdioden (LEDs).

Bei der erfindungsgemäßen Messeinheit sind mindestens einem der Lichtdetektoren 22A - 22D jeweils zwei Lichtemitter 20A, 20B bzw. 21A, 21B zugeordnet, die gleichzeitig Licht aussenden, das der mindestens eine Lichtdetektor 22A - 22D empfängt. Die beiden Lichtemitter einer Gruppe von Lichtemittern, bspw. die Lichtemitter 20A und 20B liegen sich einander gegenüber. Der zugehörige Lichtdetektor 22A - 22D ist in einer Ebene angeordnet, die mit der Ebene, in der die Lichtemitter 20A, 20B angeordnet sind, einen Winkel von 90° einschließt.

Mit der erfindungsgemäßen Messeinheit wird die Lichtausbeute verdoppelt, da das Licht von jeweils zwei Lichtemittern mit den Lichtdetektoren ausgewertet wird. Darüber hinaus wird der Einfluss unterschiedlicher Schlauchdicken innerhalb des Messkanals auf das Messergebnis reduziert, da die Messung über unterschiedliche Messstrecken erfolgt.

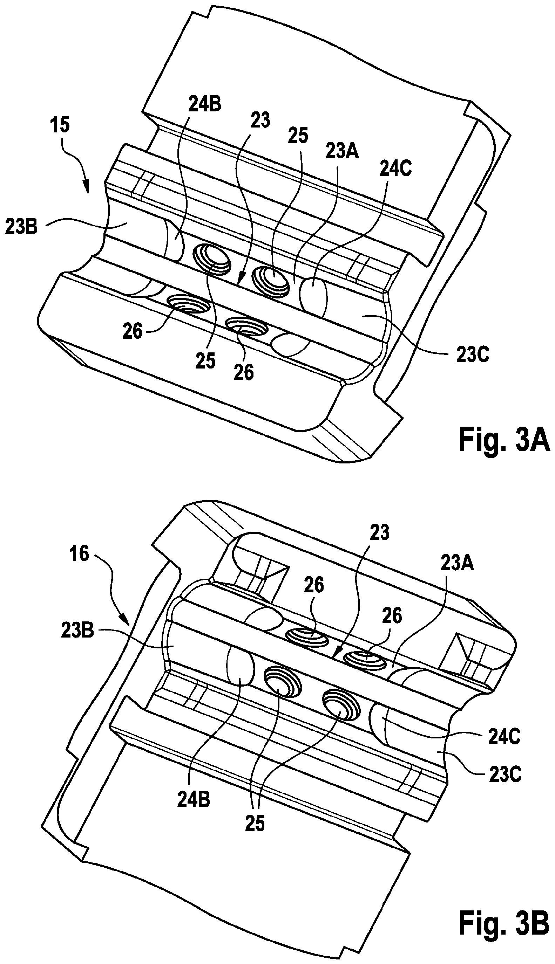

Während die Einspanneinheit 12 in den Fig. 2A und 2B nur in stark vereinfachter schematischer Darstellung gezeigt ist, zeigen die Fig. 3A und 3B in teilweise geschnittener perspektivischer Darstellung besonders bevorzugte Ausführungsformen der Aufnahmeelemente 15, 16 der Einspanneinheit 12. Die einander entsprechenden Teile sind mit den gleichen Bezugszeichen versehen.

Die beiden Aufnahmeelemente 15, 16 werden wieder mit einem in den Fig. 3A und 3B der besseren Übersichtlichkeit aber nicht dargestellten Betätigungsmechanismus bewegt, der von einem nicht dargestellten Elektromotor angetrieben wird. Diesbezüglich wird auf die schematische Darstellung der Fig. 2A und 2B verwiesen.

Die Aufnahmeelemente bilden in der geschlossenen Stellung einen Messkanal 23, der einen inneren Abschnitt 23A und zwei äußere Abschnitte 23B, 23C umfasst, die zu beiden Seiten des inneren Abschnitt 23A angeordnet sind. Innerhalb des inneren Abschnitts 23A weist der Aufnahmekanal den in den Fig. 2A und 2B gezeigten quadratischen Querschnitt auf, während der Aufnahmekanal in den äußeren Abschnitten 23B und 23C einen kreisförmigen Querschnitt hat, der dem Querschnitt der eingelegten Schlauchleitung 17 entspricht.

In dem inneren Abschnitt 23A weist jedes Aufnahmeelement 15, 16 zwei rechtwinklig zueinander stehende ebene Anlageflächen und in den äußeren Abschnitten 23B, 23C jeweils eine halbzylindrische Anlagefläche auf, die sich zu dem quadratischen bzw. kreisförmigen Messkanal in der geschlossenen Stellung der Einspanneinheit ergänzen. Zwischen den halbzylindrischen Anlageflächen der äußeren Abschnitte 23B, 23C und den beiden ebenen Anlageflächen des inneren Abschnitts 23A jedes Aufnahmeelements 15, 16 befindet sich ein Übergangsabschnitt 24B, 24C, in dem die halbzylindrischen Anlageflächen kontinuierlich in die ebenen Anlageflächen übergehen. Dadurch wird die Schlauchleitung einerseits nicht geknickt und andererseits wird die Bildung von Turbulenzen vermieden, wenn die Schlauchleitung von Blut durchströmt wird.

Die Lichtemitter 20A, 20B, 21A, 21B und die Lichtdetektoren 22A - 22C sind innerhalb des quadratischen Messkanals in den inneren Abschnitten 23A der Aufnahmeelemente 15, 16 angeordnet. Die ebenen Anlageflächen der Aufnahmeelemente weisen entsprechende Lichtaustritts- und -eintrittsöffnungen 25, 26 auf.

Zur Erkennung der Schlauchleitung 17 in der Einspanneinheit 12 weist die Vorrichtung 11 zur Bestimmung der Konzentration von Blutbestandteilen eine Überwachungseinheit 27 auf (Fig. 1). Die Überwachungseinheit 27 weist nur andeutungsweise dargestellte Mittel 27A zum Messen des Motorstroms des Elektromotors 19 auf, mit dem der Betätigungsmechanismus 18 der Einspanneinheit 12 angetrieben wird. Anstelle des Motorstroms kann aber auch eine mit dem Motorstrom korrelierende Größe, bspw. die Motorleistung gemessen werden. Darüber hinaus weist die Überwachungseinheit 27 wieder nur andeutungsweise dargestellte Mittel 27B zum Messen der Wegstrecke auf, auf der die Aufnahmeelemente 15, 16 aus der geöffneten Stellung in die geschlossene Stellung verfahren werden. Die Messwerte der Mittel 27A, 27B zum Messen des Motorstroms und der zurückgelegten Wegstrecke werden von einer Rechen- und Auswerteinheit 27C ausgewertet, die bei dem vorliegenden Ausführungsbeispiel Bestandteil der Überwachungseinheit 27 ist. Die Rechen- und Auswerteinheit 27C der Überwachungseinheit 27 ist über eine Datenleitung 28 mit der zentralen Steuereinheit 10 der extrakorporalen Blutbehandlungsvorrichtung verbunden. Sie kann aber auch Bestandteil der zentralen Steuereinheit 10 sein.

Die Rechen- und Auswerteinheit 27C der Überwachungseinheit 27 berechnet das Integral des gemessenen Motorstroms über eine Wegstrecke, die von den Aufnahmeelementen 15, 16 beim Schließen der Einspanneinheit 12 zurückgelegt wird. Das berechnete Integral des Motorstroms wird mit einem vorgegebenen Grenzwert verglichen. Wenn der Grenzwert überschritten wird, stellt die Rechen- und Auswerteinheit 27C der Überwachungseinheit 27 fest, dass eine Schlauchleitung in die Einspanneinheit 12 eingelegt ist. Ansonsten stellt die Rechen- und Auswerteinheit fest, dass eine Schlauchleitung nicht eingelegt ist. In beiden Fällen erzeugt die Rechen- und Auswerteinheit ein entsprechendes Signal, das die zentrale Steuereinheit der Blutbehandlungsvorrichtung empfängt. Im Einzelnen arbeitet die Rechen- und Steuereinheit 27C der Überwachungseinheit 27 wie folgt:

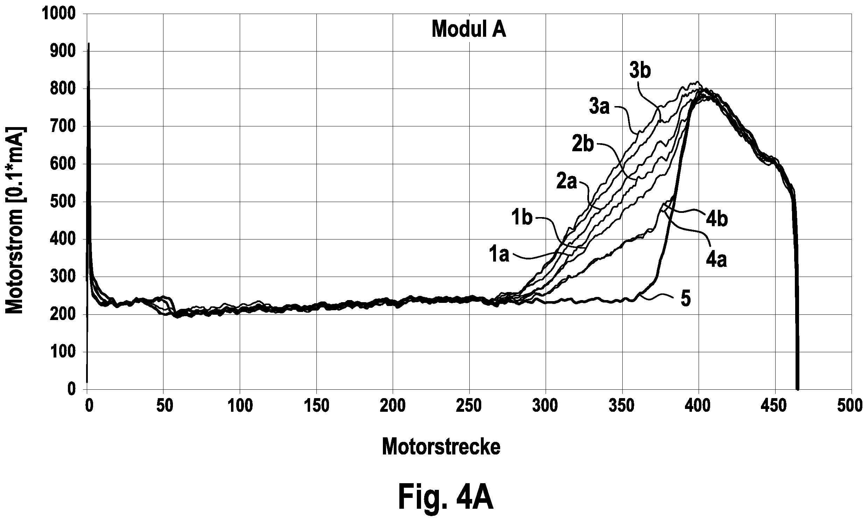

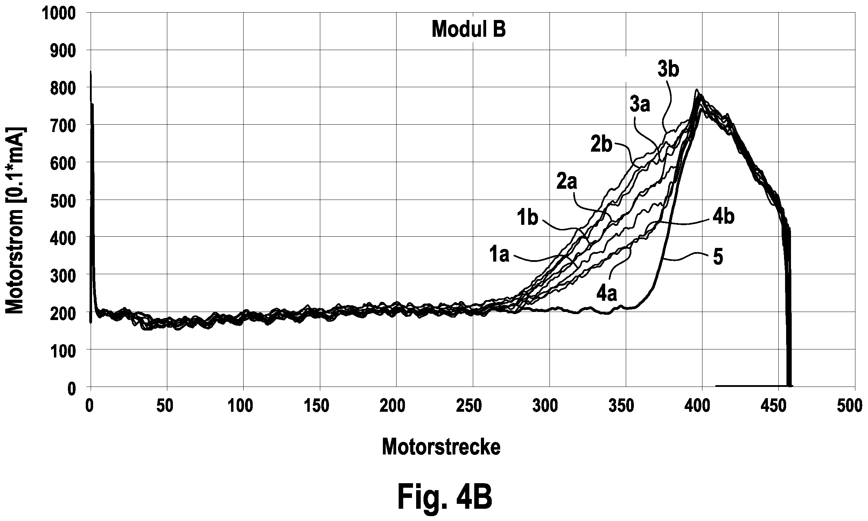

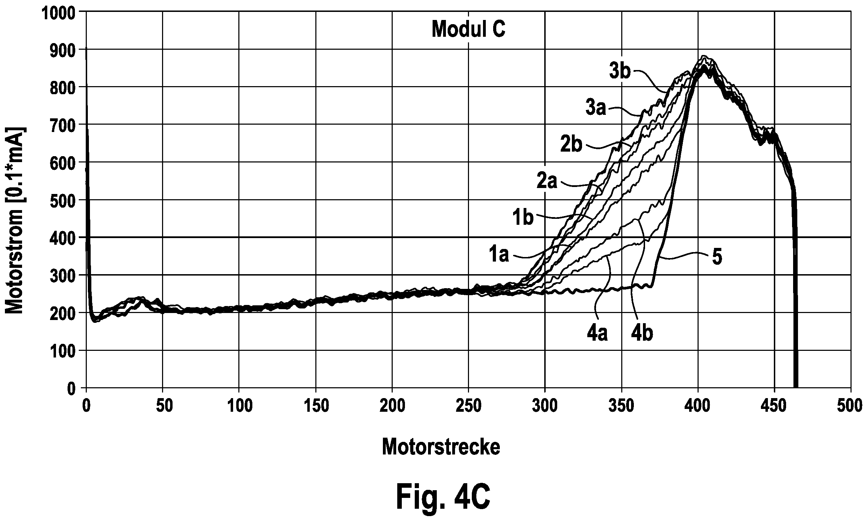

Der Verlauf des Motorstroms wird über ein bestimmtes Wegstreckenintervall überwacht. Bei dem vorliegenden Ausführungsbeispiel ist der Anfang der Wegstrecke N1 = 250 und das Ende der Wegstrecke N2 = 350 (Fig. 4A, 4B und 4C). Die Streckendifferenz zwischen den Messpunkten I(n) und I(n + 1) ist $\Delta x(n)$.

Die Rechen- und Auswerteinheit berechnet für die vorgegebene Wegstrecke zwischen N1 und N2 das Integral wie folgt:

$$A = \sum_{N1}^{N2} [I(n) - I(N1)] \cdot \Delta x(n)$$

Gleichung (1)

Die Fig. 4A, 4B und 4C zeigen den gemessenen Motorstrom [0,1 × mA] des Elektromotors zum Antrieb des Betätigungsmechanismus als Funktion der von den Aufnahmeelementen zurückgelegten Wegstrecke beim Schließen der Einspanneinheit. Die von den Aufnahmeelementen zurückgelegte Wegstrecke kann mit einer Vorrichtung erfasst werden, die pro Umdrehung des Motors eine bestimmte Anzahl von elektrischen Impulsen erzeugt. Auf der x-Achse sind hier die Anzahl der gemessenen elektrischen Impulse abgebildet, die der zurückgelegten Wegstrecke entsprechen.

Die Fig. 4A - 4C zeigen die Messergebnisse für drei verschiedene Einspanneinheiten unterschiedlicher Ausbildung, die in den Figuren als Modul A, Modul B und Modul C bezeichnet sind. In den Fig. 4A - 4C ist der Verlauf des Motorstroms beim Schließen der Einspanneinheit für vier verschiedene Schlauchleitungen dargestellt, die entweder leer sind oder mit $H_2O$ gefüllt sind. Die einzelnen Kurven sind in den Fig. 4A - 4C wie folgt bezeichnet:

5: Einspanneinheit ohne Schlauch
1a: PVC-Schlauch (Standardwandstärke) in Einspanneinheit
1b: mit $H_2O$ gefüllter PVC-Schlauch (Standardwandstärke) in Einspanneinheit
2a: PVC-Schlauch (dünne Wandstärke) in Einspanneinheit
2b: mit $H_2O$ gefüllter PVC-Schlauch (dünne Wandstärke) in Einspanneinheit
3a: PVC-Schlauch (dicke Wandstärke) in Einspanneinheit
3b: mit $H_2O$ gefüllter PVC-Schlauch (dicke Wandstärke) in Einspanneinheit
4a: leerer Schlauch (anderes Material) in Einspanneinheit
4b: mit $H_2O$ gefüllter Schlauch (anderes Material) in Einspanneinheit

Für sämtliche Messungen bei unterschiedlichen Schlauchleitungen und unterschiedlichen Einspanneinheiten zeigt sich, wenn die Einspanneinheit geschlossen wird, dass bei eingelegter Schlauchleitung der Motorstrom früher ansteigt als wenn keine Schlauchleitung eingelegt ist. Darüber hinaus zeigt sich, dass der Stromanstieg bei eingelegter Schlauchleitung stärker ist als wenn keine Schlauchleitung eingelegt ist. Folglich ist die Fläche unter den Kurven bei eingelegter Schlauchleitung größer als bei nicht eingelegter Schlauchleitung.

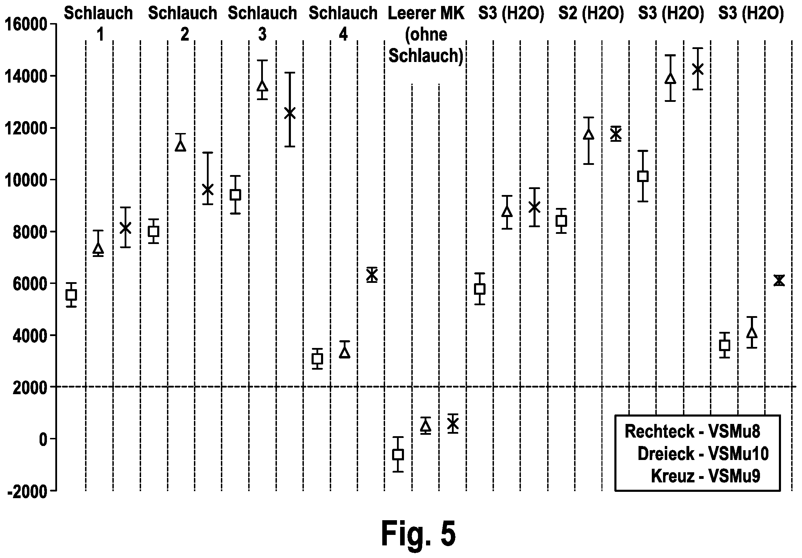

Fig. 5 zeigt die Auswertung der in den Fig. 4A - 4C dargestellten Messergebnisse. In Fig. 5 sind die Messergebnisse von Fig. 4A mit einem Rechteck, von Fig. 4B mit einem Dreieck und von Fig. 4C mit einem Kreuz dargestellt. Für die einzelnen Schlauchleitungen (PVC-Schlauch (Standardwandstärke) 1, PVC-Schlauch (dünne Wandstärke) 2, PVC-Schlauch (dicke Wandstärke) 3, Schlauch (anderes Material) 4) wurde nach Gleichung (1) das Integral berechnet. Der Wert des Integrals in 0,01 × mA × mm liegt für den PVC-Schlauch (Standardwandstärke) 1 für sämtliche Messungen mit den unterschiedlichen Einspanneinheiten sowohl für den Fall, dass der Schlauch leer ist (1a) als auch für den Fall, dass der Schlauch mit $H_2O$ gefüllt ist (1b), zwischen 4500 und 9500. Für den PVC-Schlauch (dünne Wandstärke) 2 liegt der Wert des Integrals für die Messungen zwischen 7500 und 12500, während für den PVC-Schlauch (dicke Wandstärke) 3 der Wert des Integrals zwischen 8500 und 15000 liegt. Für den Schlauch (anderes Material) 4 liegt der Wert des Integrals zwischen 2500 und 6500 deutlich niedriger. Es zeigt sich, dass der Wert des Integrals unter 2000 liegt, wenn ein Schlauch in die Einspanneinheit nicht eingelegt ist.

Bei dem vorliegenden Ausführungsbeispiel wird als vorgegebener Grenzwert für den Wert des Integrals 2000 angenommen. Damit können für sämtliche Schläuche, bei unterschiedlichen Einspanneinheiten sicher zwischen dem Fall, dass ein Schlauch in die Einspanneinheit eingelegt ist, und dem Fall, dass ein Schlauch nicht in die Einspanneinheit eingelegt ist, unterschieden werden. Wenn der Schlauch (anderes Material) nicht verwendet wird, kann der vorgegebene Grenzwert höher liegen. Am vorliegenden Ausführungsbeispiel wird ein Grenzwert von 3000 festgelegt.

Für die Messung des Blutvolumens können Blutschläuche aus PVC oder PUR oder anderen Materialien verwendet werden. In der Praxis stellt sich daher nicht nur das Problem der Erkennung eines Blutschlauchs in der Einspanneinheit der Vorrichtung zur Bestimmung der Konzentration eines Blutbestandteils, sondern auch der Identifizierung des in die Einspanneinheit eingelegten Blutschlauchtyps.

Es hat sich in der Praxis gezeigt, dass das Material, aus dem der Blutschlauch besteht, einen Einfluss auf die Genauigkeit der Messung der Konzentration eines Blutbestandteils haben kann. Daher erlaubt die Identifizierung des Blutschlauchs, auch auf die Genauigkeit der Messung zu schließen. So ist es möglich, einen akustischen und/oder optischen Hinweis auf die Messgenauigkeit zu geben. Die Messung kann in Abhängigkeit von dem verwendeten Blutschlauch auch korrigiert oder sogar verhindert werden.

Im Folgenden wird eine Rechen- und Auswerteinheit der Überwachungseinheit beschrieben, die neben der Erkennung des Blutschlauchs auch die Identifizierung des Blutschlauchtyps erlaubt.

Mit einem Blutschlauch aus PUR, der sich durch eine geringe Toleranz der Wandstärke auszeichnet, wird in der Praxis eine hohe Messgenauigkeit erzielt. Ein Schlauch aus PUR zeichnet sich im Vergleich zu einem Schlauch aus PVC durch eine größere Härte und eine höhere Elastizität aus. Der PUR-Schlauch übt somit eine größere Rückstellkraft auf die Aufnahmeelemente der Aufnahmeeinheit aus.

Versuche haben gezeigt, dass sich eine Unterscheidung zwischen einem Blutschlauch aus PUR und PVC mit dem erfindungsgemäßen Verfahren bzw. der erfindungsgemäßen Vorrichtung mit besonders hoher Sicherheit dann treffen lässt, wenn der Blutschlauch vor der Messung für eine vorbestimmte Zeitdauer zwischen den Aufnahmeelementen vorverpresst wird.

Die erfindungsgemäße Vorrichtung und das erfindungsgemäße Verfahren sehen daher vor, dass nach dem Einlegen der Schlauchleitung in die Einspanneinheit 12 der Betätigungsmechanismus 18 zum Schließen der Aufnahmeelemente 15, 16 in Betrieb gesetzt wird, wobei die Aufnahmeelemente solange geschlossen werden, bis eine vorbestimmte Anpresskraft auf die Schlauchleitung ausgeübt wird, und nach einem vorbestimmten Zeitintervall die Aufnahmeelemente wieder geöffnet werden. Zur Durchführung der eigentlichen Messung werden die Aufnahmeelemente dann wieder geschlossen. Dieser Prozess kann von der Überwachungseinheit 27 vollautomatisch gesteuert werden.

Nachfolgend wird ein Ausführungsbeispiel beschrieben, bei dem eine Kalibrierung zur Erhöhung der Messgenauigkeit vorgesehen ist.

Das Verhältnis von Strom und Drehmoment wird im Allgemeinen durch die folgende Gleichung beschrieben:

$$I_n(m) = k_n \cdot m$$

wobei *m* das Drehmoment und *k* eine Konstante und *n* eine Bezeichnung (Nummer) der bei der Messung verwendeten Einspanneinheit ist.

Es hat sich gezeigt, dass sich die einzelnen Einspanneinheiten der gleichen Bauart aufgrund von Toleranzen des Betätigungsmechanismus im Schließverhalten voneinander unterscheiden können.

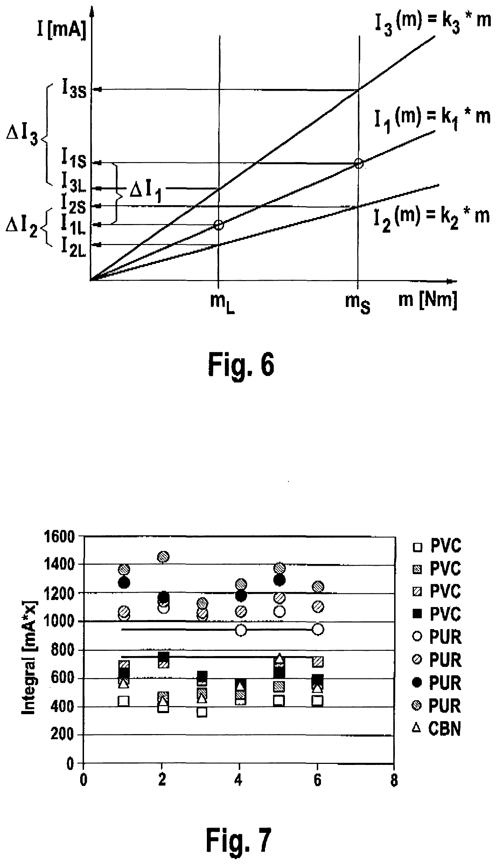

Fig. 6 zeigt den Motorstrom $I$ [mA], der von den Mitteln 27A zum Messen des Motorstroms gemessen wird, in Abhängigkeit von dem Drehmoment m [Nm], der von dem Elektromotor 19 aufgebracht wird. Das Verhältnis von Strom und Drehmoment $I_1(m)$, $I_2(m)$, $I_3(m)$ ist für drei verschiedene Einspanneinheiten 15 dargestellt. Der Kreis bei $m_L$ zeigt den Arbeitspunkt der Leerlaufmessung, bei der der Schlauch nicht in die Einspanneinheit eingelegt ist, und der Kreis bei $m_S$ den Arbeitspunkt der Schlauchpressung, bei der der Schlauch in die Einspanneinheit eingelegt ist. Mit steigendem k-Wert nimmt der Stromunterschied $\Delta I_n$ zwischen der Leerlaufmessung $\Delta I_{nL}$ und der Schlauchpressung $\Delta I_{nS}$ zu ($\Delta I_3$ > $\Delta I_1$ > $\Delta I_2$). Daraus ergibt sich, dass für die Eliminierung des Einflusses der Toleranzen der Einspanneinheit auf die Messung nicht ausreichend ist, ein nur bei einer Leerlaufmessung ermitteltes Offset zu berücksichtigen. Daher wird eine 1-Punkt-Kalibrierung mit einem Kalibrierfaktor k vorgenommen.

Die Überwachungseinheit 27 ist derart ausgebildet, dass eine Messung mit der folgenden Kalibrierung vorgenommen wird. Sämtliche Rechenoperationen können in einem nicht dargestellten Mikroprozessor ausgeführt werden, der Teil der Überwachungseinheit 27 bzw. der Rechen- und Auswerteinheit 27C ist. Hierzu ist der Mikroprozessor der Rechen- und Auswerteinheit 27C für die Durchführung der folgenden Rechenoperationen programmiert.

Zunächst werden die Aufnahmeelemente 15, 16 der Einspanneinheit 27 elektromotorisch geöffnet, sofern sie nicht schon offen sind, um eine Leerlaufmessung, bei der eine Schlauchleitung nicht eingelegt wird, durchführen zu können. Daraufhin werden die Aufnahmeelemente 15, 16 zur Durchführung der Leerlaufmessung elektromotorisch geschlossen, wobei der Motorstrom $I_L(x)$ mit den Mitteln (27A) zum Messen des Motorstroms gemessen wird. Die Messung zur Ermittlung des Leerlaufstroms braucht nicht vor jeder Blutbehandlung durchgeführt zu werden. Es ist ausreichend, wenn die Messung in vorgegebenen Zeitabständen, beispielsweise in einem Abstand von einem Monat durchgeführt wird. Mit der Leerlaufmessung wird ein inhärenter Systemeinfluss eliminiert.

Um die Zuverlässigkeit der Kalibrierung zu erhöhen, kann vorgesehen sein, die Leerlaufmessung einige Male zu wiederholen. Der Verlauf des Motorstroms wird hierbei integriert und die Integralwerte werden über die Anzahl der Messungen gemittelt.

Daraufhin werden die Aufnahmeelemente 15, 16 der Einspanneinheit 12 wieder aufgefahren, um die Schlauchleitung einlegen zu können. Vor der eigentlichen Messung mit Schlauchpressung erfolgt vorzugsweise eine Vorverpressung der Schlauchleitung.

Zur Vorverpressung der Schlauchleitung werden die Aufnahmeelemente 15, 16 bei eingelegter Schlauchleitung um eine vorgegebene Wegstrecke x zugefahren, um einen vorbestimmten Anpressdruck auf die Schlauchleitung auszuüben, der von Material sowie Durchmesser und Wandstärke der Schlauchleitung abhängig ist. Die Vorverpressung der Schlauchleitung erfolgt vorzugsweise während des Befüll- oder Spülvorgangs der Blutbehandlungsvorrichtung. In dieser Phase ist die Schlauchleitung mit einer Flüssigkeit befüllt, die eine vorgegebene Temperatur hat. Während des Befüll- oder Spülvorgangs ist die Temperatur der Spülflüssigkeit beispielsweise 36°, wobei die Flussrate der Flüssigkeit beispielsweise 400 ml/min ist. Die Zeitdauer der Vorpressung kann 3 min betragen. Diese Werte haben sich für die Vorverpressung in der Praxis als besonders vorteilhaft erwiesen.

Nach der Vorverpressung werden die Aufnahmeelemente 15, 16 zur Durchführung der eigentlichen Messung wieder aufgefahren und geschlossen, um den Motorstrom $I_S$ (x) bei Schlauchpressung in Abhängigkeit von der Wegstrecke x zu messen.

Anschließend wird der Motorstrom $I(x)$ nach der folgenden Gleichung berechnet:

$$I(\mathrm{x}) = I_S(\mathrm{x}) - I_L(\mathrm{x}) - I_0 \qquad\qquad \text{Gleichung (2)}$$

Nunmehr wird das Integral nach der folgenden Gleichung berechnet:

$$A = k \cdot \int_{x1}^{x2} [I_S(x) - I_L(x) - I_0]\, dx$$

$$\text{Gleichung (3)}$$

wobei k ein Kalibrierfaktor ist, $x_1$ und $x_2$ die Bedingung $I(x_1) \geq 0$ und $I(x_2) \geq 0$ erfüllen und $I_0$ ein vorgegebener Strom, beispielsweise 5 mA ist.

Zur Bestimmung des Kalibrierfaktors k wird folgende Kalibrierung vorgenommen. Für die Kalibrierung wird in mehreren Messungen mit mehreren Einspanneinheiten der gleichen Bauart ein Referenzwert für das Integral $A_{Ref}$ berechnet, wobei zur Berechnung des Referenzwertes der Mittelwert der ermittelten Integrale $A_n$ berechnet wird. Darüber hinaus wird für die zu kalibrierende Einspanneinheit 12 das Integral $A_0$ berechnet.

Der Kalibrierfaktor k wird dann nach der folgenden Gleichung berechnet:

$$k = \frac{A_{Ref}}{A_0}$$

$$\text{Gleichung (4)}$$

Das nach Gleichung (3) berechnete Integral A wird in der Rechen- und Auswerteinheit 27C mit einem vorgegebenen Grenzwert verglichen. Hierzu weist die Rechen- und Auswerteinheit 27C eine nicht dargestellte Vergleichseinheit auf.

Wenn das berechnete Integral A größer als der vorgegebene Grenzwert ist, wird festgestellt, dass eine Schlauchleitung eines ersten Typs eingelegt ist, die Schlauchleitung beispielsweise ein PUR-Schlauch ist. Ist das berechnete Integral hingegen kleiner als der vorgegebene Grenzwert, wird festgestellt, eine Schlauchleitung eines zweiten Typs eingelegt ist, die Schlauchleitung beispielsweise ein PVC-Schlauch ist.

Eine bevorzugte Ausführungsform sieht vor, dass eine Aussage über die Materialeigenschaften dann nicht getroffen wird, wenn das berechnete Integral A in einem Wertebereich liegt, der zwischen einem vorgegebenen ersten Grenzwert, beispielsweise für einen PUR-Schlauch, und einem vorgegebenen zweiten Grenzwert, beispielsweise für einen PVC-Schlauch liegt, wobei der erste Grenzwert für den PUR-Schlauch größer als der zweite Grenzwert für den PVC-Schlauch ist. Ein PUR-Schlauch wird also identifiziert, wenn das Integral größer als der obere erste Grenzwert ist, während ein PVC-Schlauch identifiziert wird, wenn das Integral kleiner als der untere zweite Grenzwert ist.

Wenn die Rechen- und Auswerteinheit 27C festgestellt hat, dass die Schlauchleitung ein PUR-Schlauch oder ein PVC-Schlauch ist, wird ein erstes oder zweites Steuersignal erzeugt. Die Art der verwendeten Schlauchleitung kann beispielsweise auf einer nicht dargestellten Anzeigeeinheit der Überwachungseinheit 27 dargestellt werden. Auch kann auf der Anzeigeeinheit angezeigt werden, wenn das Integral A kleiner als der obere erste Grenzwert und größer als der zweite untere Grenzwert ist, d.h. in dem oben genannten Wertebereich liegt, so dass eine Bestimmung der Art der verwendeten Schlauchleitung nicht mit hoher Sicherheit möglich ist.

In Versuchen hat sich gezeigt, dass sich mit dem Vergleich des Integrals mit einem oberen und unteren Grenzwert der Schlauchleitungstyp sicher identifizieren lässt. Zur Identifikation eines Schlauchleitungstyps kann alternativ auch der Maximalwert $I_{max}$ des gemessenen Stroms bestimmt werden, wobei der Maximalwert des Stroms $I_{max}$ mit einem Grenzwert verglichen wird. Liegt der Maximalwert $I_{max}$ des gemessenen Stroms oberhalb des Grenzwertes wird auf den einen Schlauchleitungstyp, beispielsweise den PUR-Schlauch, und liegt der Maximalwert $I_{max}$ des gemessenen Stroms unterhalb des Grenzwertes wird auf den anderen Schlauchleitungstyp, beispielsweise den PUR-Schlauch, geschlossen. Auch kann die Steilheit der Flanke des Stromanstiegs mit einem Grenzwert verglichen werden, um den Schlauchleitungstyp zu identifizieren. Auch diese Operationen können wieder von der Vergleichseinheit ausgeführt werden.

Es ist auch möglich, die Bestimmung der Schlauchleitung auf der Grundlage einer Kombination von zwei oder drei der oben genannten Größen vorzunehmen, um die Sicherheit weiter zu erhöhen. Dann schließt die Überwachungseinheit auf eine bestimmte Schlauchleitung nur dann, wenn bei einer Auswertung auf der Grundlage von zwei Größen oder auf der Grundlage sämtlicher Größen auf einen bestimmten Schlauchleitungstyp geschlossen wird.

Bei der ersten alternativen Ausführungsform sieht die Auswerteinheit vor, auf einen PUR-Schlauch zu schließen, wenn der Maximalwert des Stroms $I_{max}$ größer als ein vorgegebener Grenzwert ist, während auf einen PVC-Schlauch geschlossen wird, wenn der Maximalwert des Stroms $I_{max}$ kleiner als der vorgegebene Grenzwert ist.

Bei der zweiten alternativen Ausführungsform sieht die Auswerteinheit vor, auf einen PUR-Schlauch zu schließen, wenn die Steilheit der Flanke des Stromanstieges, d.h. die Ableitung der Funktion in dem Bereich der Stromanstiegsflanke, größer als ein vorgegebener Grenzwert ist, während auf einen PVC-Schlauch geschlossen wird, wenn die Steilheit der Flanke des Stromanstieges kleiner als ein vorgegebener Grenzwert ist.

Fig. 7 zeigt die Messergebnisse für sechs Einspanneinheiten 1 bis 6 der gleichen Bauart und vier unterschiedliche PVC-Schläuche und vier unterschiedliche PUR-Schläuche sowie einen weiteren Schlauch CBN aus einem anderen Material, wobei die PVC-Schläuche in Fig. 7 mit Rechtecken und die PUR-Schläuche mit Kreisen gekennzeichnet sind. Der Wertebereich, in dem eine sichere Identifikation des Schlauchtyps nicht gewährleistet ist, liegt zwischen dem oberen Grenzwert, der in Fig. 7 mit der oberen waagerechten Linie (Grenzwert (PUR)) gekennzeichnet ist, und dem unteren Grenzwert, der mit der unteren Linie (Grenzwert (PVC)) gekennzeichnet ist.

Der Sicherheitsabstand zwischen der oberen und unteren Linie berechnet sich aus dem Quotienten aus der Differenz des oberen Grenzwertes (PUR) und des unteren Grenzwertes (PVC) und dem Mittelwert der Grenzwerte.

Das oben beschriebene Messverfahren wurde mit vier Einspanneinheiten A, B, C, D getestet, wobei ein dünner PUR-Schlauch mit einer geringen Wandstärke, ein dicker PUR-Schlauch mit einer großen Wandstärke und ein Standard-Schlauch aus PVC verwendet wurde. Für den Test wurde der Schlauch mit Wasser gefüllt, das eine Temperatur von 36,6° hat. Das Wasser würde durch die Schläuche gepumpt.

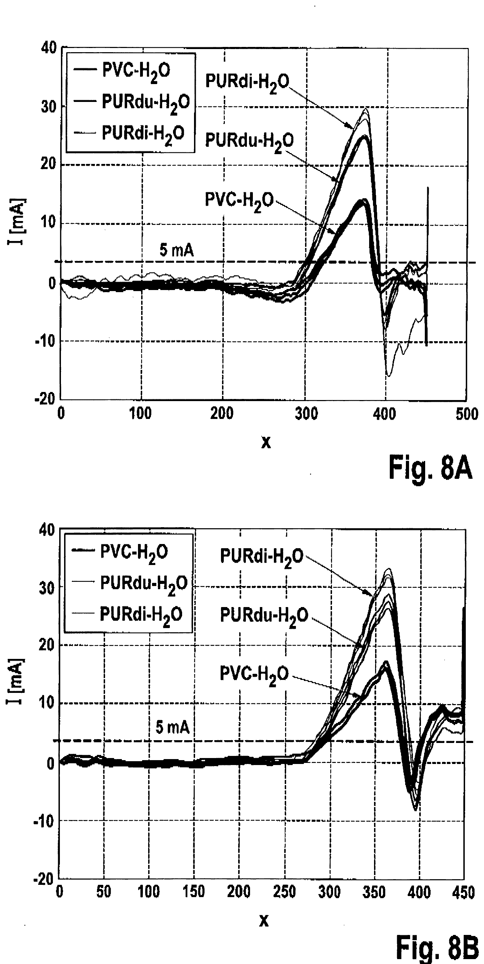

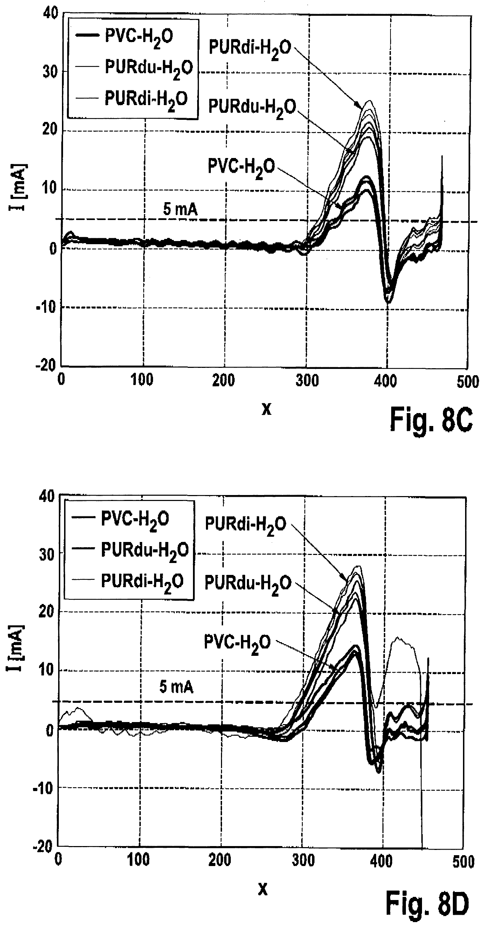

Die Figuren 8A bis 8D zeigen den über 10 Punkte geglätteten Motorstrom I [mA] als Funktion der Motorstrecke x für die einzelnen Einspanneinheiten A, B, C, D, wobei der dünne PUR-Schlauch mit einer geringen Wandstärke mit PURdu, der dicke PUR-Schlauch mit einer großen Wandstärke mit PURdi und der Standard-PVC-Schlauch mit PVC bezeichnet ist. Der vorgegebene konstante Strom $I_0$ ist 5 mA.

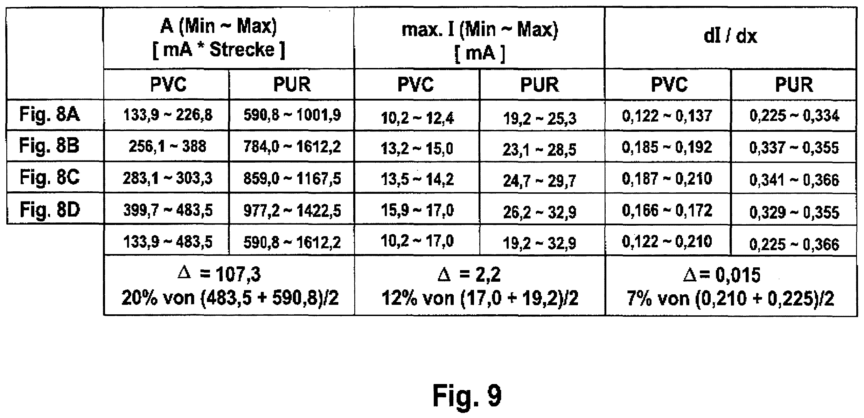

Fig. 9 zeigt eine Tabelle, in der das Integral A, der maximale Strom $I_{max}$ und die Steigung der Flanke $dI/dx$ für die einzelnen Einspanneinheiten A, B, C, D und die unterschiedlichen Schlauchtypen PVC und PUR eingetragen ist. Der Sicherheitsanstand ist in der Tabelle mit Δ bezeichnet.

Es zeigt sich, dass sich die Wertebereiche die unterschiedlichen Auswertmethoden auf der Grundlage des Integrals A, des maximalen Stroms $I_{max}$ und der Steigung der Flanke $dI/dx$ nicht überschneiden. Folglich lassen sich die einzelnen Schlauchtypen eindeutig identifizieren. Die höchste Sicherheit bei der Identifizierung des Schlauchtyps zeigt sich aber bei der Auswertung auf der Grundlage des Integrals.

## Patentansprüche

1. Vorrichtung zum Einspannen einer Schlauchleitung, insbesondere einer Schlauchleitung eines extrakorporalen Blutkreislaufs einer extrakorporalen Blutbehandlungsvorrichtung, mit einer Einspanneinheit (12), die ein erstes Aufnahmeelement (15) und ein zweites Aufnahmeelement (16) zum Einspannen der Schlauchleitung aufweist, wobei die Einspanneinheit (12) einen Betätigungsmechanismus (18) aufweist, der derart ausgebildet ist, dass unter Aufbringung einer Spannkraft das erste und zweite Aufnahmeelement aus einer ersten die Schlauchleitung freigebenden Stellung in eine zweite die Schlauchleitung einspannende Stellung gegeneinander bewegbar sind, und dass der Betätigungsmechanismus (18) einen Elektromotor zum Antrieb des Betätigungsmechanismus aufweist,
**dadurch gekennzeichnet, dass**
die Vorrichtung eine Überwachungseinheit (27) aufweist, die derart ausgebildet ist, dass eine in die Aufnahmeelemente (15, 16) eingelegte Schlauchleitung erkennbar ist, wobei die Überwachungseinheit (27) Mittel (27A) zum Messen des Motorstroms des Elektromotors zum Antrieb des Betätigungsmechanismus der Einspanneinheit oder einer mit dem Motorstrom korrelierenden Größe und eine Rechen- und Auswerteinheit (27C) aufweist, die derart ausgebildet ist, dass auf der Grundlage der Veränderung des gemessenen Motorstroms oder der mit dem Motorstrom korrelierenden Größe eine in die Aufnahmeelemente (15, 16) eingelegte Schlauchleitung erkennbar ist.

2. Vorrichtung nach Anspruch 1, **dadurch gekennzeichnet, dass** die Überwachungseinheit (27) derart ausgebildet ist, dass der mit den Mitteln (27A) zum Messen des Motorstroms gemessene Motorstrom mit einem Korrekturwert korrigiert wird, der bei einer Kalibriermessung gewonnen wird, wenn die Schlauchleitung nicht in die Einspanneinheit eingelegt ist.

3. Vorrichtung nach Anspruch 1 oder 2, **dadurch gekennzeichnet, dass** die Überwachungseinheit (27) Mittel (27B) zum Messen der von den Aufnahmeelementen (15, 16) zurückgelegten Wegstrecke aufweist und die Rechen- und Auswerteinheit (27C) der Überwachungseinheit (27) derart ausgebildet ist, dass der Motorstrom in Abhängigkeit von der von den Aufnahmeelementen zurückgelegten Wegstrecke ausgewertet wird.

4. Vorrichtung nach Anspruch 3, **dadurch gekennzeichnet, dass** die Rechen- und Auswerteinheit (27C) der Überwachungseinheit (27) derart ausgebildet ist, dass das Integral des Motorstroms und/oder der Maximalwert des Motorstroms über eine vorgegebene von den Aufnahmeelementen (15, 16) zurückgelegte Wegstrecke mit einem vorgegebenen Grenzwert verglichen wird, wobei bei Überschreiten des Grenzwertes darauf geschlossen wird, dass in die Aufnahmeelemente eine Schlauchleitung eingelegt ist, oder
das Integral des Motorstroms und/oder der Maximalwert des Motorstroms über eine vorgegebene von den Aufnahmeelementen (15, 16) zurückgelegte Wegstrecke mit einem vorgegebenen Grenzwert verglichen wird, wobei bei Überschreiten des Grenzwertes darauf geschlossen wird, dass in die Aufnahmeelemente eine bestimmte erste Schlauchleitung eingelegt ist und bei Unterschreiten des Grenzwertes darauf geschlossen wird, dass in die Aufnahmeelemente eine bestimmte zweite Schlauchleitung eingelegt ist, wobei die erste und zweite Schlauchleitung unterschiedliche Schlauchleitungen sind.

5. Vorrichtung nach einem der Ansprüche 1 bis 4, **dadurch gekennzeichnet, dass** die Aufnahmeelemente (15, 16) der Einspanneinheit (12) derart ausgebildet sind, dass die zwischen dem ersten und zweiten Aufnahmeelement eingespannte Schlauchleitung einen quadratischen Querschnitt hat.

6. Vorrichtung nach einem der Ansprüche 1 bis 5, **dadurch gekennzeichnet, dass** das erste Aufnahmeelement (15) der Einspanneinheit (12) zwei rechtwinklig zueinander stehende ebene Anlageflächen (15A, 15B) und das zweite Aufnahmeelement (16) der Einspanneinheit (12) zwei rechtwinklig zueinander stehende ebene Anlageflächen (16A, 16B) aufweist, wobei das erste und zweite Aufnahmeelement (15, 16) auf einer Achse (a) gegeneinander bewegbar sind, die mit den ebenen Anlageflächen (15A, 15B; 16A, 16B) des ersten und zweiten Aufnahmeelements einen Winkel von 45° einschließt.

7. Vorrichtung nach einem der Ansprüche 1 bis 6, **dadurch gekennzeichnet, dass** das erste Aufnahmeelement (15) zu beiden Seiten der ebenen Anlageflächen (15A, 15B; 23A) halbzylindrische Anlageflächen (23B, 23C) und das zweite Aufnahmeelement (16) zu beiden Seiten der ebenen Anlageflächen (16A, 16B; 23A) halbzylindrische Anlageflächen (23B, 23C) aufweist.

8. Vorrichtung nach Anspruch 7, **dadurch gekennzeichnet, dass** das erste Aufnahmeelement (15) zu beiden Seiten der ebenen Anlageflächen (15A, 15B; 23A) einen Übergangsabschnitt (24B, 24C) aufweist, der derart ausgebildet ist, dass die inneren ebenen Anlageflächen (23A) kontinuierlich in die äußeren halbzylindrischen Anlageflächen (24B, 24C) übergehen.

9. Vorrichtung nach einem der Ansprüche 1 bis 8, **dadurch gekennzeichnet, dass** die Messeinheit (13) Lichtemitter (20A, 20B; 21A, 21B) und Lichtdetektoren (22A bis 22D) aufweist, die in dem ersten und zweiten Aufnahmeelement (15, 16) angeordnet sind.

10. Vorrichtung nach Anspruch 9, **dadurch gekennzeichnet, dass** die ebenen Anlageflächen (23A) der Aufnahmeelemente (15, 16) den Lichtemittern (20A, 20B; 21A, 21B) und Lichtdetektoren (22A bis 22D) zugeordnete Lichtaustritts- und -eintrittsöffnungen (25, 26) aufweisen.

11. Vorrichtung nach Anspruch 9 oder 10, **dadurch gekennzeichnet, dass** mindestens einem Lichtdetektor (22A bis 22D) mindestens eine Gruppe von zwei Lichtemittern (20A, 20B; 21A, 21B) zugeordnet ist.

12. Vorrichtung nach Anspruch 11, **dadurch gekennzeichnet, dass** die zwei Lichtemitter der mindestens einen Gruppe von Lichtemittern (20A, 20B; 21A, 21B) auf einander gegenüberliegenden Seiten angeordnet sind.

13. Vorrichtung nach einem der Ansprüche 1 bis 12, **dadurch gekennzeichnet, dass** die Vorrichtung eine Vorrichtung zur Bestimmung der Konzentration eines Bestandteils von Blut in einer Schlauchleitung ist, wobei die Vorrichtung aufweist:

eine Messeinheit (13) zum Einkoppeln von elektromechanischer Strahlung durch die Schlauchleitung in das Blut und Messen der durch die Schlauchleitung aus dem Blut austretenden elektromagnetischen Strahlung, und eine mit der Messeinheit (13) zusammenwirkende Rechen- und Auswerteinheit (14) zum Bestimmen der Konzentration eines Bestandteils von Blut.

14. Verfahren zur Erkennung einer Schlauchleitung, insbesondere einer Schlauchleitung eines extrakorporalen Blutkreislaufs einer extrakorporalen Blutbehandlungsvorrichtung, in einer Einspanneinheit (12), wobei die Einspanneinheit aufweist:

ein erstes Aufnahmeelement (15) und ein zweites Aufnahmeelement (16) zum Einspannen der Schlauchleitung, und

einen Betätigungsmechanismus (18), der derart ausgebildet ist, dass unter Aufbringung einer Spannkraft das erste und zweite Aufnahmeelement aus einer ersten die Schlauchleitung frei gebenden Stellung in eine zweite die Schlauchleitung einspannende Stellung gegeneinander bewegbar sind, wobei der Betätigungsmechanismus (18) einen Elektromotor (19) zum Antrieb des Betätigungsmechanismus aufweist,
**dadurch gekennzeichnet, dass**
der Motorstrom des Elektromotors zum Antrieb des Betätigungsmechanismus der Einspanneinheit oder einer mit dem Motorstrom korrelierenden Größe gemessen wird, wobei auf der Grundlage der Veränderung des gemessenen Motorstroms oder der mit dem Motorstrom korrelierenden Größe die in die Aufnahmeelemente eingelegte Schlauchleitung erkannt wird.

15. Verfahren nach Anspruch 14, **dadurch gekennzeichnet, dass** der gemessene Motorstrom mit einem Korrekturwert korrigiert wird, der bei einer Kalibriermessung gewonnen wird, wenn die Schlauchleitung nicht in die Einspanneinheit eingelegt ist.

16. Verfahren nach Anspruch 14 oder 15, **dadurch gekennzeichnet, dass** die von den Aufnahmeelementen (15, 16) zurückgelegte Wegstrecke gemessen wird und der Motorstrom in Abhängigkeit von der von den Aufnahmeelementen zurückgelegten Wegstrecke ausgewertet wird.

17. Verfahren nach Anspruch 16, **dadurch gekennzeichnet, dass**

das Integral des Motorstroms und/oder der Maximalwert des Motorstroms über eine vorgegebene von den Aufnahmeelementen zurückgelegte Wegstrecke mit einem vorgegebenen Grenzwert verglichen wird, wobei bei Überschreiten des Grenzwertes darauf geschlossen wird, dass in die Aufnahmeelemente eine Schlauchleitung eingelegt ist, oder
das Integral des Motorstroms und/oder der Maximalwert des Motorstroms über eine vorgegebene von den Aufnahmeelementen zurückgelegte Wegstrecke mit einem vorgegebenen Grenzwert verglichen wird, wobei bei Überschreiten des Grenzwertes darauf geschlossen wird, dass in die Aufnahmeelemente eine bestimmte erste Schlauchleitung eingelegt ist und bei Unterschreiten des Grenzwertes darauf geschlossen wird, dass in die Aufnahmeelemente eine bestimmte zweite Schlauchleitung eingelegt ist, wobei die erste und zweite Schlauchleitung unterschiedliche Schlauchleitungen sind.

18. Verfahren nach einem der Ansprüche 14 bis 17, **dadurch gekennzeichnet, dass** die Einspanneinheit (12) eine Einspanneinheit einer Vorrichtung zur Bestimmung der Konzentration eines Bestandteils von Blut ist.

**Claims**

1. A device for tensioning a hose line, in particular a hose line of an extracorporeal blood circuit of an extracorporeal blood treatment apparatus, comprising

a clamping unit (12), which comprises a first receiving element (15) and a second receiving element (16) for clamping the hose line, the clamping unit (12) comprising an actuation mechanism (18) which is constituted such that, when a clamping force is applied, the first and second receiving element can be moved towards one another from a first position releasing the hose line into a second position clamping the hose line, and that the actuation mechanism (18) comprises an electric motor for driving the actuation mechanism

**characterised in that**

the device comprises a monitoring unit (27) which is configured such that a hose line inserted into the receiving elements (15, 16) can be detected,

the monitoring unit (27) comprises means (27A) for measuring the motor current of the electric motor for driving the actuation mechanism of the clamping unit or a variable correlating with the motor current and a computing and evaluation unit (27C), which is configured such that a hose line inserted into the receiving elements (15, 16) can be detected on the basis of the change in the measured motor current or the variable correlating with the motor current.

2. The device according to claim 1, **characterised in that** the monitoring unit (27) is configured such that the motor current measured with the means (27A) for measuring the motor current is corrected with a correction value being obtained in a calibration measurement, when the hose line is not inserted into the clamping unit.

3. The device according to claim 1 or 2, **characterised in that** the monitoring unit (27) comprises means (27B) for measuring the path covered by the receiving elements (15, 16) and the computing and evaluation unit (27C) of the monitoring unit (27) is configured such that the motor current is evaluated as a function of the path covered by the receiving elements.

4. The device according to claim 3, **characterised in that** the computing and evaluation unit (27C) of the monitoring unit (27) is configured such that

the integral of the motor current and/or the maximum value of the motor current over a preset path covered by the receiving elements (15, 16) is compared with a preset threshold value, it being concluded that a hose line is inserted into the receiving elements when the threshold value is exceeded, or

the integral of the motor current and/or the maximum value of the motor current over a preset path covered by the receiving elements (15, 16) is compared with a preset threshold value, it being concluded that a specific first hose line is inserted into the receiving elements when the threshold value is exceeded and a specific second hose line is inserted into the receiving elements when the threshold value is fallen below, the first and second hose line being different hose lines.

5. The device according to any one of claims 1 to 4, **characterised in that** the receiving elements (15, 16) of the clamping unit (12) are constituted such that the hose line clamped between the first and the second receiving element has a square cross-section.

6. The device according to any one of claims 1 to 5, **characterised in that** the first receiving element (15) of the clamping unit (15) comprises two plane contact faces (15A, 15B) standing at right angles to one another and the second receiving element (16) of the clamping unit (15) comprises two plane contact faces (16A, 16B) standing at right angles to one another, wherein the first and second receiving element (15, 16) can be moved towards one another on an axis (a) which forms an angle of 45° with the plane contact faces (15A, 15B; 16A, 16B) of the first and second receiving element.

7. The device according to any one of claims 1 to 6, **characterised in that** the first receiving element (15) comprises semi-cylindrical contact faces (23B, 23C) on both sides of the plane contact faces (15A, 15B; 23A) and the second receiving element (16) comprises semi-cylindrical contact faces (23B, 23C) on both sides of the plane contact faces (16A, 16B; 23A).

8. The device according to claim 7, **characterised in that** the first receiving element (15) comprises a transition section (24B, 24C) on both sides of the plane contact faces (15A, 15B; 23A), said transition section being constituted such that the inner plane contact faces (23A) are transformed continuously into the outer semi-cylindrical contact faces (24B, 24C).

9. The device according to any one of claims 1 to 8, **characterised in that** the measurement unit (13) comprises light emitters (20A, 20B; 21A, 21B) and light detectors (22A to 22D), which are disposed in the first and second receiving element (15, 16).

10. The device according to claim 9, **characterised in that** the plane contact faces (23A) of the receiving element (15, 16) comprise light outlet and inlet openings (25, 26) assigned to light emitters (20A, 20B; 21A, 21B) and light detectors (22A to 22D).

11. The device according to claim 9 or 10, **characterised in that** at least one group of two light emitters (20A, 20B; 21A, 21B) is assigned to at least one light detector (22A to 22D).

12. The device according to claim 11, **characterised in that** the two light emitters of the at least one group of light emitters (20A, 20B; 21A, 21B) are disposed on mutually opposite sides.

13. The device according to any one of claims 1 to 12, **characterised in that** the device is a device for determining the concentration of a blood constituent in a hose line, wherein the device comprises:

a measuring unit (13) for coupling of electromagnetic radiation through the hose line into the blood and for measuring the electromagnetic radiation emerging through the hose line from the blood, and
a computing and evaluation unit (14) for determining the concentration of a blood constituent, said computing and evaluation unit cooperating with the measurement unit (13).

14. A method for detecting a hose line, in particular a hose line of an extracorporeal blood circuit of an extracorporeal blood treatment apparatus, in a clamping unit (12), wherein the clamping unit comprises:

a first receiving element (15) and a second receiving element (16) for clamping the hose line, and
an actuation mechanism (18) which is constituted such that, when a clamping force is applied, the first and second receiving element can be moved towards one another from a first position releasing the hose line into a second position clamping the hose line, wherein the actuation mechanism (18) comprises an electric motor (19) for driving the actuation mechanism,
**characterised in that**
the motor current of the electric motor for driving the actuation mechanism of the clamping unit or a variable correlating with the motor current is measured, wherein the hose line inserted into the receiving elements is detected on the basis of the change in the measured motor current or the variable correlating with the motor current.

15. The method according to claim 14, **characterised in that** the motor current is corrected with a correction value being obtained in a calibration measurement, when the hose line is not inserted into the clamping unit.

16. The method according to claim 14 or 15, **characterised in that** the path covered by the receiving elements (15, 16) is measured and the motor current is evaluated as a function of the path covered by the receiving elements.

17. The method according to claim 16, **characterised in that**

the integral of the motor current and/or the maximum value of the motor current over a preset path covered by the receiving elements is compared with a preset threshold value, wherein it is concluded that a hose line is inserted into the receiving elements when the threshold value is exceeded, or
the integral of the motor current and/or the maximum value of the motor current over a preset path covered by the receiving elements is compared with a preset threshold value, it being concluded that a specific first hose line is inserted into the receiving elements when the threshold value is exceeded and a specific second hose line is inserted into the receiving elements when the threshold value is fallen below, the first and second hose line being different hose lines.

18. Method according to any one of claims 14 to 17, **characterised in that** the clamping unit (12) is a clamping unit of a device for determining the concentration of a blood constituent.

**Revendications**

1. Dispositif pour le serrage d'une conduite flexible, plus particulièrement d'une conduite flexible d'un circuit sanguin extracorporel d'un dispositif de traitement de sang extracorporel, avec une unité de serrage (12), qui comprend un premier élément de logement (15) et une deuxième élément de logement (16) pour le serrage de la conduite flexible, dans lequel l'unité de serrage (12) comprend un mécanisme d'actionnement (18) qui est conçu de façon à ce que, en appliquant une force de serrage, les premier et deuxième éléments de logement l'un contre l'autre peuvent être déplacés d'une première position libérant la conduite flexible vers une deuxième position serrant la conduite flexible et de façon à ce que le mécanisme d'actionnement (18) comprend un moteur électrique pour l'entraînement du mécanisme d'actionnement,
**caractérisé en ce que**
le dispositif comprend une unité de surveillance (27) qui est conçue de façon à ce qu'une conduite flexible insérée dans les éléments de logement (15, 16) puisse être détectée, dans lequel l'unité de surveillance (27) comprend des moyens (27A) pour la mesure du courant de moteur du moteur électrique pour l'entraînement du mécanisme d'actionnement de l'unité de serrage ou d'une grandeur corrélée avec le courant de moteur et comprend une unité de calcul et d'analyse (27C), qui est conçue de façon à ce que, sur la base de la variation du courant de moteur mesuré ou de grandeur corrélée avec le courant de moteur, une conduite flexible insérée dans les éléments de logement (15, 16) puisse être détectée.

2. Dispositif selon la revendication 1, **caractérisé en ce que** l'unité de surveillance (27) est conçue de façon à ce que le courant de moteur mesuré avec les moyens (27A) pour la mesure du courant de moteur est corrigé avec une valeur de correction qui est obtenue lors d'une mesure de calibrage, lorsque la conduite flexible n'est pas insérée dans l'unité de serrage.

3. Dispositif selon la revendication 1 ou 2, **caractérisé en ce que** l'unité de surveillance (27) comprend des moyens (27B) pour la mesure du trajet parcouru par les éléments de logement (15, 16) et l'unité de calcul et d'analyse (27C) de l'unité de surveillance (27) est conçue de façon à ce que le courant de moteur soit analysé en fonction du trajet parcouru par les éléments de logement.

4. Dispositif selon la revendication 3, **caractérisé en ce que** l'unité de calcul et d'analyse (27C) de l'unité de surveillance (27) est conçue de façon à ce que

l'intégrale du courant de moteur et/ou la valeur maximale du courant de moteur sur un trajet prédéterminé parcouru par les éléments de logement (15, 16) soit comparée avec une valeur limite prédéterminée, dans lequel, lors d'un dépassement de la valeur limite, on en déduit qu'une conduite flexible est insérée dans les éléments de logement ou
l'intégrale du courant de moteur et/ou la valeur maximale du courant de moteur sur un trajet prédéterminé parcouru par les éléments de logement (15, 16) soit comparée avec une valeur limite prédéterminée, dans lequel, lors d'un dépassement de la valeur limite, on en déduit qu'une première conduite flexible déterminée est insérée dans les éléments de logement et, lors du passage en dessous de la valeur limite, on en déduit qu'une deuxième conduite flexible déterminée est insérée dans les éléments de logement, dans lequel les première et deuxième conduites flexibles sont des conduites flexibles différentes.

5. Dispositif selon l'une des revendications 1 à 4, **caractérisé en ce que** les éléments de logement (15, 16) de l'unité de serrage (12) sont conçus de façon à ce que la conduite flexible serrée dans les premier et deuxième éléments de logement présente une section transversale carrée.

6. Dispositif selon l'une des revendications 1 à 5, **caractérisé en ce que** le premier élément de logement (15) de l'unité de serrage (12) comprend deux surfaces d'appui planes (15A, 15B) perpendiculaires entre elles et le deuxième élément de logement (16) de l'unité de serrage (12) comprend deux surfaces d'appui planes (16A, 16B) perpendiculaires entre elles, dans lequel les premier et deuxième éléments de logement (15, 16) sont mobiles l'un contre l'autre sur un axe (a) qui forme, avec les surfaces d'appui planes (15A, 15B ; 16A, 16B) du premier et du deuxième élément de logement, un angle de 45°.

7. Dispositif selon l'une des revendications 1 à 6, **caractérisé en ce que** le premier élément de logement (15) comprend, des deux côtés des surfaces d'appui planes (15A, 15B ; 23A), des surfaces d'appui semi-cylindriques (23B, 23C) et le deuxième élément de logement (16) comprend, des deux côtés des surfaces d'appui planes (16A, 16B ; 23A), des surfaces d'appui semi-cylindriques (23B, 23C).

8. Dispositif selon la revendication 7, **caractérisé en ce que** le premier élément de logement (15) comprend, des deux côtés des surfaces d'appui planes (15A, 15B ; 23A), une portion de transition (24B, 24C) qui est conçue de façon à ce que les surfaces d'appui planes internes (23A) deviennent progressivement les surfaces d'appui semi-cylindriques externes (24B, 24C).

**9.** Dispositif selon l'une des revendications 1 à 8, **caractérisé en ce que** l'unité de mesure (13) comprend des émetteurs de lumière (20A, 20B ; 21A, 21B) et des détecteurs de lumière (22A à 22D) qui sont disposés dans les premier et deuxième éléments de logement (15, 16).

**10.** Dispositif selon la revendication 9, **caractérisé en ce que** les surfaces d'appui planes (23A) des éléments de logement (15, 16) comprennent des ouvertures de sortie et d'entrée de lumière (25, 26) correspondant aux émetteurs de lumière (20A, 20B ; 21A, 21B) et aux détecteurs de lumière (22A à 22D).

**11.** Dispositif selon la revendication 9 ou 10, **caractérisé en ce qu'**à au moins un détecteur de lumière (22A à 22D) correspond au moins un groupe de deux émetteurs de lumière (20A, 20B ; 21A, 21B).

**12.** Dispositif selon la revendication 11, **caractérisé en ce que** les deux émetteurs de lumière (20A, 20B ; 21A, 21B) sont disposés sur des côtés opposés entre eux.

**13.** Dispositif selon l'une des revendications 1 à 12, **caractérisé en ce que** le dispositif comprend un dispositif de détermination de la concentration d'un composant du sang dans une conduite flexible, dans lequel ce dispositif comprend :

une unité de mesure (13) pour l'entrée d'un rayonnement électromagnétique dans le sang à travers la conduite flexible et la mesure du rayonnement électromagnétique sortant du sang à travers la conduite flexible et une unité de calcul et d'analyse (14) interagissant avec l'unité de mesure (13) pour la détermination de la concentration d'un composant du sang.

**14.** Procédé de détection d'une conduite flexible, plus particulièrement d'une conduite flexible d'un circuit sanguin extracorporel d'un dispositif de traitement de sang extracorporel, dans une unité de serrage (12), dans lequel l'unité de serrage comprend :

un premier élément de logement (15) et un deuxième élément de logement (16) pour le serrage de la conduite flexible et un mécanisme d'actionnement (18) qui est conçu de façon à ce que, en appliquant une force de serrage, les premier et deuxième éléments de logement peuvent être déplacés d'une première position libérant la conduite flexible vers une deuxième position serrant la conduite flexible, dans lequel le mécanisme d'actionnement (18) comprend un moteur électrique (19) pour l'entraînement du mécanisme d'actionnement, **caractérisé en ce que** le courant de moteur du moteur électrique pour l'entraînement du mécanisme d'actionnement de l'unité de serrage ou une grandeur corrélée avec le courant de moteur est mesurée, dans lequel, sur la base de la variation du courant de moteur mesuré ou de la grandeur corrélée avec le courant de moteur, la conduite flexible insérée dans les éléments de logement est détectée.

**15.** Procédé selon la revendication 14, **caractérisé en ce que** le courant de moteur mesuré est corrigé avec une valeur de correction qui est obtenue lors d'une mesure de calibrage, lorsque la conduite flexible n'est pas insérée dans l'unité de serrage.

**16.** Procédé selon la revendication 14 ou 15, **caractérisé en ce que** le trajet parcouru par les éléments de logement (15, 16) est mesuré et le courant de moteur est analysé en fonction du trajet parcouru par les éléments de logement.

**17.** Procédé selon la revendication 16, **caractérisé en ce que**

l'intégrale du courant de moteur et/ou la valeur maximale du courant de moteur sur un trajet prédéterminé parcouru par les éléments de logement est comparée avec une valeur limite prédéterminée, dans lequel, lors d'un dépassement de la valeur limite, on en déduit qu'une conduite flexible est insérée dans les éléments de logement ou l'intégrale du courant de moteur et/ou la valeur maximale du courant de moteur sur un trajet prédéterminé parcouru par les éléments de logement est comparée avec une valeur limite prédéterminée, dans lequel, lors d'un dépassement de la valeur limite, on en déduit qu'une première conduite flexible déterminée est insérée dans les éléments de logement et, lors du passage en dessous de la valeur limite, on en déduit qu'une deuxième conduite flexible déterminée est insérée dans les éléments de logement, dans lequel les première et deuxième conduites flexibles sont des conduites flexibles différentes.

**18.** Procédé selon l'une des revendications 14 à 17, **caractérisé en ce que** l'unité de serrage (12) est une unité de serrage d'un dispositif pour la détermination de la concentration d'un composant du sang.

**Fig. 1**

**Fig. 2A**

**Fig. 2B**

**Fig. 3A**

**Fig. 3B**

Fig. 4A

EP 2 984 988 B1

Fig. 4B

EP 2 984 988 B1

Modul C

Fig. 4C

Motorstrecke

Motorstrom [0.1*mA]

3b
3a
2b
2a
1b
1a
4a
4b
5

Fig. 5

EP 2 984 988 B1

**Fig. 6**

**Fig. 7**

Fig. 8A

Fig. 8B

Fig. 8C

Fig. 8D

| | A (Min ~ Max) [ mA * Strecke ] | | max. I (Min ~ Max) [ mA ] | | dI / dx | |
|---|---|---|---|---|---|---|
| | PVC | PUR | PVC | PUR | PVC | PUR |
| Fig. 8A | 133,9 ~ 226,8 | 590,8 ~ 1001,9 | 10,2 ~ 12,4 | 19,2 ~ 25,3 | 0,122 ~ 0,137 | 0,225 ~ 0,334 |
| Fig. 8B | 256,1 ~ 388 | 784,0 ~ 1612,2 | 13,2 ~ 15,0 | 23,1 ~ 28,5 | 0,185 ~ 0,192 | 0,337 ~ 0,355 |
| Fig. 8C | 283,1 ~ 303,3 | 859,0 ~ 1167,5 | 13,5 ~ 14,2 | 24,7 ~ 29,7 | 0,187 ~ 0,210 | 0,341 ~ 0,366 |
| Fig. 8D | 399,7 ~ 483,5 | 977,2 ~ 1422,5 | 15,9 ~ 17,0 | 26,2 ~ 32,9 | 0,166 ~ 0,172 | 0,329 ~ 0,355 |
| | 133,9 ~ 483,5 | 590,8 ~ 1612,2 | 10,2 ~ 17,0 | 19,2 ~ 32,9 | 0,122 ~ 0,210 | 0,225 ~ 0,366 |
| | $\Delta$ = 107,3 20% von (483,5 + 590,8)/2 | | $\Delta$ = 2,2 12% von (17,0 + 19,2)/2 | | $\Delta$ = 0,015 7% von (0,210 + 0,225)/2 | |

**Fig. 9**

EP 2 984 988 B1

**IN DER BESCHREIBUNG AUFGEFÜHRTE DOKUMENTE**

**In der Beschreibung aufgeführte Patentdokumente**

- WO 200800433 A1
- EP 1579196 B1
- US 5372136 A